# EUROPEAN PATENT SPECIFICATION

(11) **EP 2 086 426 B1**
(45) Date of publication and mention of the grant of the patent: **31.07.2013**
(21) Application number: 07864225.3
(22) Date of filing: 09.11.2007
(51) Int. Cl.: A61B 17/11

(54) **RING MAGNETS FOR SURGICAL PROCEDURES**
RINGMAGNETEN FÜR CHIRURGISCHE VERFAHREN
AIMANTS TORIQUES DESTINÉS À DES INTERVENTIONS CHIRURGICALES

(30) Priority: 10.11.2006 US 857901 P
(43) Date of publication of application: 12.08.2009
(73) Proprietor: Cook Medical Technologies LLC, Bloomington, IN 47404 (US)
(72) Inventor: KENNEDY, Kenneth C., Clemmons, NC 27012 (US); CHANG, Kenneth J., Cerritos, CA 90703 (US)
(74) Representative: Kroher, Jürgen
(86) International application number: PCT/US2007/084299
(87) International publication number: WO 2008/061024

(56) References cited:
- EP-A- 1 493 391
- EP-A- 1 894 514
- WO-A-81/00668
- US-A- 5 690 656

## Description

### FIELD OF THE INVENTION

The present invention relates generally to devices for forming an anastomosis between two viscera, and more particularly relates to such devices employing magnets.

### BACKGROUND. OF THE INVENTION

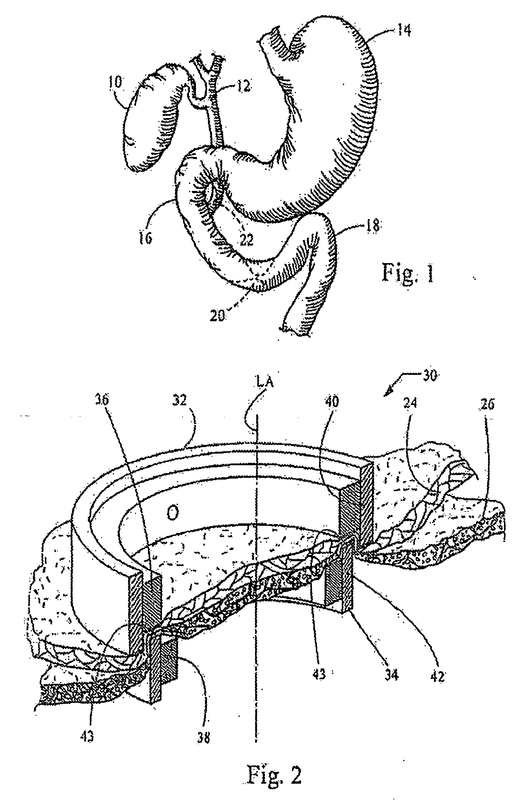

Historically, gastro-intestinal (GI) surgery has been performed to create a channel between two viscera for the purpose of redirecting bodily fluids, i.e. an anastomosis. For example, intestinal contents or bile may be redirected in patients who have developed an obstruction of the bowel or bile duct due to such conditions as tumors, ulcers, inflammatory strictures or trauma. With reference to FIG. 1, the relative positions of several organs of the abdominal cavity are shown, including the gall bladder 10, the bile duct 12, the stomach 14, the duodenum 16 and the jejunum 18 of the small intestine. Inflammatory strictures 20, 22 of the jejunum 18 and bile duct 12 are shown by the dotted lines in FIG. 1. It will be recognized that there may be a need to anastamose many different viscera, such as the jejunum18 and the stomach 14 (gastrojejunostomy), the bile duct 12 and the duodenum 16, two sections of the small or large intestines (not shown), or various other combinations of viscera such as during bariatric surgery.

During surgery to form an anastomosis, the two tissues are often brought together and affixed to one another using fixators such as sutures, staples, or some other fixation means. While fixators are being placed, the tissues of the respective viscera are held in proximity to one another using various means. In open surgery this is usually accomplished with graspers, forceps, or other tissue holding instruments that are manipulated by clinicians. In laparoscopic surgery, similar instruments may be used, except that the laparotic access limits the number of instruments to a few percutaneous "ports," making the technical challenge of the procedure much greater.

When these types of GI surgery are performed, there exists the potential to breach the mural boundary. Thus, extreme care must be taken to prevent contamination of the pleural and abdominal cavities with GI contents, which are laden with bacteria that do not naturally occur in those locations. If significant contamination occurs, then serious infection can set in, which can lead to serious illness or death if not treated early and vigorously,

To address these limitations and minimize the Invasiveness of such surgeries, magnetic anastomosis devices (MADs) have been developed for forming anastomosis. An exemplary MAD is disclosed in U.S. Patent No. 5,690,656, from which the preamble of claim 1 derives. Generally, the MAD of the '656 patent includes first and second magnet assemblies comprising magnetic cores which are surrounded by thin metal rims. The first and second magnet assemblies are positioned in the two viscera between which the anastomosis is desired and brought into close proximity to each other. Due to the magnetic attraction between the two magnetic cores, the walls of two adjacent viscera are compressed between the magnet assemblies and in particular the metal rims, resulting in ischemic necrosis of the walls to produce an anastomosis between the two viscera.

MADs may be delivered through surgical intervention such as laparotomy, over a wire guide using a pushing catheter (and typically under fluoroscopy), by simply swallowing the magnet assemblies of the MAD and using massage under fluoroscopy to align the two magnet assemblies, or endoscopically using grasping forceps. Within about ten days the visceral tissues surrounding the magnets fuse together, and the magnets and entrapped necrotic tissue subsequently detach from the surrounding tissue to leave an opening between the viscera. The detached magnet pair may pass through the remainder of the GI tract naturally, and uneventfully, but are preferably grasped with forceps (ideally just prior to complete detachment) and removed during a follow-up endoscopic procedure.

### BRIEF SUMMARY OF THE INVENTION

The present invention provides a medical device for rapidly forming an anastomosis between two viscera, while reducing the technical challenge and minimizing the potential of breaching the mural boundary. In this manner, the anastomosis may be formed with surety before the patient leaves the medical facility and eliminates the need for a follow-up procedure. An additional protection against breach of the mural bounding is also provided and there is minimal risk of the anastomosis becoming separated or forming a leak while the patient is not in the medical facility.

According to the present invention, a medical device for approximating the tissues of two viscera includes a first magnet assembly and a second magnet assembly. Each of the magnet assemblies includes a magnetic core defining an axial opening, a jacket and a transverse passageway. The axial openings are sized to permit formation of the anastomosis therein. The transverse passageway is sized to permit passage of a wire guide therethrough for placement of the first and second magnet assemblies. The first and second magnet assemblies define first and second rims projecting axially from the first and second jackets beyond the first and second magnetic cores. The first and second magnet assemblies define first and second rims positioned proximate the first and second axial openings. Once the tissues have been approximated with the medical device, tissue excision and affixation may be performed, preferably endoscopically.

According to more detailed aspects of the medical device, the first and second axial openings may be substantially the same size or may be different in size. The first and second rims may be sized to nest within each other. The first and second rims preferably define atraumatic engagement surfaces such as flat or rounded edges.

A method for forming an anastomosis between two viscera is also provided. Generally, a pair of magnet assemblies is provided having a construction as described above. One magnet assembly is placed into one viscera and the other magnet assembly is placed into the other viscera such that the pair of magnet assemblies are magnetically attracted and compress the tissue of the two viscera between the magnet assemblies. A portion of the tissue of the two viscera located within the axial openings is excised. The tissues of the two viscera are affixed together, thereby forming a secure anastamosis.

The placing step preferably includes introducing a wire guide into one of the viscera and translating one of the magnet assemblies along the wire guide into the viscera. The excising step includes introducing a cutting instrument into one of the viscera and manipulating the cutting instrument. The cutting instrument may be an electrosurgical device, although numerous other cutting instruments may be employed. The excising step may be performed endoscopically, and the cutting instrument may be introduced through a working channel of an endoscope. The affixing step includes affixing together the tissue of the two viscera located within the axial openings, such as by suturing or stapling. Preferably, the affixing step is also performed endoscopically.

### BRIEF DESCRIPTION OF THE DRAWINGS

The accompanying drawings incorporated in and forming a part of the specification illustrate several aspects of the present invention, and together with the description serve to explain the principles of the invention. In the drawings:

FIG. 1 depicts various abdominal organs and anatomy where the medical device may be employed ;

FIG. 2 is a perspective view, cut to reveal a cross-section, of a medical device for forming an anastomosis ; partly according to the present invention;

FIG. 3 is a cross-sectional view of the medical device depicted in FIG. 2;

FIG. 4 is a cross-sectional view similar to FIG. 3 showing the step of excising tissue;

FIGS. 5 and 5a are cross-sectional and perspective views, respectively, showing the step of suturing the tissue; and

FIG. 6 is a cross-sectional view of another medical device for forming an anastomosis fully in accordance with the teachings of the present invention.

### DETAILED DESCRIPTION OF THE INVENTION

According to the invention, embodiment in FIGS. 2-5 must be modified to locate the axially projecting rims defined the flanges at a position closer to the axial openings 44, 46, thereby moving the area of approximated tissue closer to the area of excised tissue.

Turning now to the figures, FIG. 2 depicts a medical device 30 having a pair of magnet assemblies 32, 34. The first magnet assembly 32 includes a first magnetic core 36 and a first jacket 40 connected to the outer periphery of the first magnetic core 36. The first jacket 40 projects axially, (i.e., relative to the longitudinal axis LA), beyond the first magnetic core 36 to define a rim sized to receive the second magnet assembly 34. The second magnetic assembly 34 includes a second magnetic core 38 having a second jacket 42 connected to the outer periphery thereof. Preferably the first and second magnetic assemblies 32 and 34 are disc shaped, although any annular configuration may be employed. The second jacket 42 is sized smaller (i.e., has a smaller outer diameter) than the first jacket 40 such that the pair of magnet assemblies 32, 34 are capable of nesting. Specifically, the first and second magnet cores 36, 38 have their respective poles oriented such that they are magnetically attracted to one another (when in proximity to one another) to form the nested configuration of the medical device 30 depicted in FIG. 2. In this configuration, the tissue 24, 26 from the two viscera are compressed between the first and second magnet assemblies 32, 34, thereby approximating the tissues 24, 26 so that formation of an anastomosis may be accomplished. The jackets 40, 42 define engagement surfaces 43, and in this embodiment the engagement surface 43 of the jacket 42 presses the tissue 24, 26 against the first magnetic core 36. As such, the engagement surfaces 43 are atraumatically designed. The surfaces 43 are depicted as flat in the figures, although numerous atraumatic shapes may be employed as will be recognized by those skilled in the art, such as rounded edges or the like.

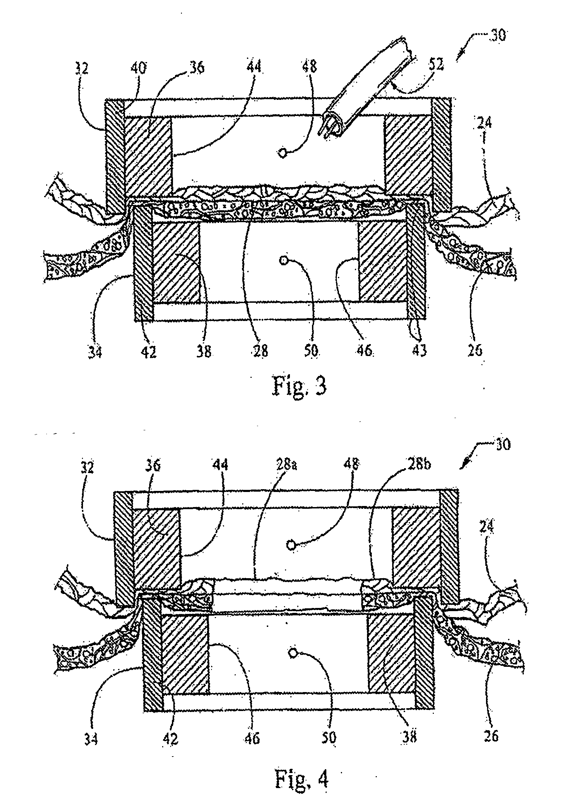

Turning now to FIG. 3, the first and second magnetic cores 36, 38 define large axial openings 44, 46, respectively. These large openings 44, 46 provide access to the tissue portions 28 held between the first and second magnet assemblies 32, 34, for the introduction of a cutting device 52. The axial openings are thus sized to correspond with the desired size of the anastomosis and are preferably at least 10 mm for gastrointestinal anastomoses. Each of the magnet assemblies 32, 34 includes a smaller transverse passageway 48, 50 (respectively) extending generally orthogonal to the longitudinal axis LA. The transverse passageways 48, 50 are much smaller in size than the axial openings 44, 46, and are generally sized to receive a wire guide therethrough for introduction of the magnet assemblies 32, 34 into the desired viscera over the wire guide, as will be discussed below.

A method of forming an anastomosis employing the magnetic assemblies 32, 34 of the medical device 30 will now be described with reference to FIGS. 3-5. Placement of the magnet assemblies 32, 34 may be accomplished in many ways, such as by use of a wire guide over which the assemblies are traversed by way of the transverse passageways 48, 50 in combination with a pushing catheter, or by swallowing the magnet assembly 32, 34 and employing massage under fluoroscopy, or through the use of endoscopic procedures using implements such as grasping forceps, by way of laparoscopic or open surgery, or other controlled insertion through natural orifices.

As one example, gastrojejunostomy is typically performed by delivery of a first (jejunal) magnet assembly (e.g. magnet assembly 34) over a wire guide and through the oral cavity. A pushing catheter (not shown) is used to push the jejunal magnet assembly along the wire guide to a point within the jejunum 16 that is adjacent to the stomach wall 14. This is usually accomplished with the aid of dynamic radiographic imaging (fluoroscopy). A second (gastric) magnet assembly (e.g. magnet assembly 32) is then Introduced into the stomach using an endoscope and grasping forceps. When the gastric magnet is manipulated into a position that is near the jejunal magnet, the force of attraction between the magnets increases to a level that is sufficient to approximate the jejunal and stomach walls, thereby compressing these tissues together between the two magnet assemblies, as shown in FIG. 3.

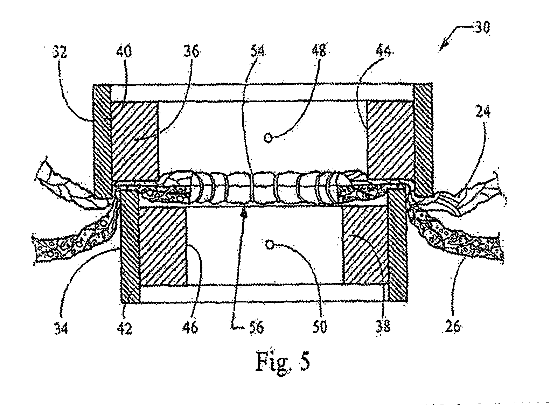

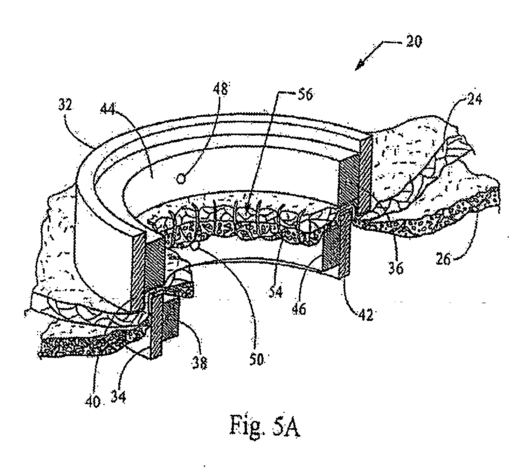

As shown in FIG. 4, a portion of the compressed tissue 28 is then excised using the cutting device 52 (FIG. 3) which is manipulated around the large axial opening (44 or 46). This leaves an excised edge 28a. Preferably, the cutting device 52 is an electrosurgical instrument which is employed through a working channel of an endoscope, although it will be recognized by those skilled In the art that numerous types of cutting devices may be employed with or without the aid of an endoscope, such as puncturing and dilating devices. Upon removal of the excised portion of the tissue, the remaining portion 28b of the tissues 24, 26 are affixed together as shown in FIGS. 5 and 5a. As with the excision, the affixing of the tissues 24, 26 is preferably accomplished endoscopically by using, for example, an affixation device such as a suturing Instrument to place one or more sutures 54, although It will be recognized by those skilled in the art that numerous types of affixing devices may be used with or without the aid of an endoscope, such as clips, staples, adhesives, or mechanical closure devices. Upon completion of the affixation, a secure anastomosis 56 is formed in the tissues 24, 26 between the two viscera.

Removal of the magnet assemblies 32, 34 may be accomplished by traditional methods used with MADs. For example, grasping forceps can be used to take a hold of one of the magnet assemblies 32, 34 and withdraw the magnet assembly from the site. The second magnet may then pass naturally through the body, or may also be removed endoscopically using grasping forces. The second grasping forceps may be used to engage the other magnet assembly to provide sufficient traction to overcome the magnetic force. Finally, it will be recognized that the magnet assemblies 32, 34 could be left in place to cause necrosis of the remaining portion 28b of the tissues 24, 26, thereby forming an even larger anastomosis than the initial anastomosis 56. In this case, the jackets 40, 42 may be provided with sharp engagement surfaces 43 to facilitate the necrosis of tissues 24, 26, although this is not necessary. After formation of the larger anastomosis, the magnet assemblies 32, 34 may be removed manually or naturally or a combination thereof.

It will be recognized by those skilled in the art that during these anastomosis formation procedures, the ring of compression of the tissues 24, 26 provides an additional barrier that guards against leakage of the GI contents or other bodily fluids depending on the viscera involved. Likewise, the anastomosis is formed with surety before the patient leaves the medical facility, eliminating the need for a follow-up procedure. It will be noted that In the prior embodiment of FIGS. 2-5, the first and second axial openings 44, 46 were generally of a different size and diameter, but they can also have a substantially similar size, (i.e., diameter). The similar size of the openings allows the user to determine the maximum area of tissue that can be removed from either side of the device.

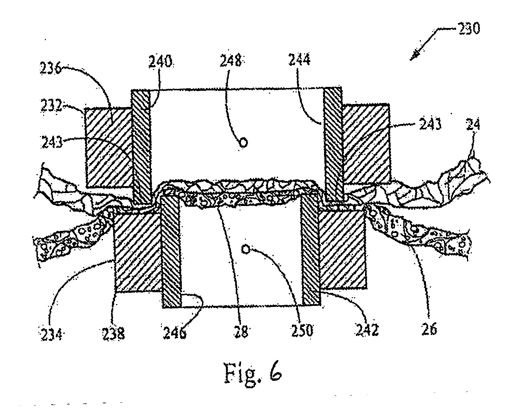

In the embodiment of FIG. 6, the magnetic device 230 includes a pair of magnet assemblies 232, 234 having magnetic cores 236, 238 and jackets 240, 242. In this embodiment, the Jackets 240, 242 are already shown connected to the inner periphery of the magnetic cores 236, 238which is in accordance with the invention. As such, the jackets 240, 242 define the large axial openings 244, 246. It will be recognized that the engagement surface 243 of jacket 242 is positioned closer to the tissue portion 28 to be excised and affixed.

The foregoing description of various embodiments of the invention has been presented for purposes of illustration and description. It is not intended to be exhaustive or to limit the invention to the precise embodiments disclosed. For example, the medical device can be applied in many different situations other than GI surgery, for example in forming anastomosis between vascular structures, or any body structures. Numerous modifications or variations are possible in light of the above teachings. The embodiments discussed were chosen and described to provide the best illustration of the principles of the invention and its practical application to thereby enable one of ordinary skill in the art to utilize the invention in various embodiments and with various modifications as are suited to the particular use contemplated.

## Claims

1. A medical device (30, 230) for approximating tissues (24, 26) of two viscera for formation of an anastomosis using a surgical instrument (52), the medical device comprising:
a first magnet assembly (32, 232) having a first magnetic core (36, 236) defining a first axial opening (44, 244), and a first jacket (40,240);
a second magnet assembly (34, 234) having a second magnetic core (38, 238) defining a second axial opening (46, 246), and a second jacket (42,242);
at least one of the first (32, 232) and second (34, 234) magnet assemblies having a transverse passageway (48, 50, 248, 250) sized to receive and permit passage of a wire guide therethrough for placement of the first (32, 232) and second (34, 234) magnet assemblies;
wherein the first (32, 232) and second (34, 234) magnet assemblies define first , and second rims projecting axially from the first (40,240) and second (42,242) jackets beyond the first (36, 236) and second (38, 238) magnetic cores;
**characterized in that**
the first (44, 244) and second (46, 246) axial openings are sized to receive the surgical instrument (52) and permit formation of the anastomosis therein, and that
the first and second rims are positioned proximate the first (44, 244) and second (46, 246) axial openings of the first (36, 236) and second (38, 238) magnetic cores.

2. The medical device (30, 230) of claim 1, wherein the first (44, 244) and second (46, 246) axial openings are substantially the same size.

3. The medical device (30, 230) of claim 1, wherein the first (44, 244) and second (46, 246) axial openings are different in size.

4. The medical device (30, 230) of claim 3, wherein the first (40, 240) and second (42, 242) rims are sized to nest within each other.

5. The medical device (30, 230) of claim 1, wherein the first and second rims define atraumatic engagement surfaces (43,243)

6. The medical device (30, 230) of claim 1, wherein the first (32, 232) and second (34, 234) magnet assemblies are disc shaped.

7. The medical device (30, 230) of claim 1, wherein the first (44, 244) and second (46, 246) axial openings have a diameter of at least 10 mm.

## Patentansprüche

1. Medizinische Vorrichtung (30, 230) zum gegenseitigen Näherbringen von Gewebe (24, 26) von zwei Viszera zum Bilden einer Anastomose unter Verwendung eines chirurgischen Instruments (52), wobei die medizinische Vorrichtung umfasst:
eine erste magnetische Baugruppe (32, 232), welche einen ersten magnetischen Kern (36, 236), der eine erste axiale Öffnung (44, 244) definiert, und einen ersten Mantel (40, 240) aufweist;
eine zweite magnetische Baugruppe (34, 234), welche einen zweiten magnetischen Kern (38, 238), der eine zweite axiale Öffnung (46, 246) definiert, und einen zweiten Mantel (42, 242) aufweist;
wobei mindestens eine aus der ersten (32, 232) und der zweiten (34, 234) magnetischen Baugruppe einen transversalen Durchgang (48, 50, 248, 250) aufweist, welcher derart dimensioniert ist, um Abschnitte einer Drahtführung darin aufzunehmen und deren Durchtritt zuzulassen, um die erste (32, 232) und die zweite (34, 234) magnetische Baugruppe zu platzieren;
wobei die erste (32, 232) und die zweite (34, 234) magnetische Baugruppe einen ersten und einen zweiten Rand definieren, welche axial von dem ersten (40, 240) und von dem zweiten (42, 242) Mantel über den ersten (36, 236) oder den zweiten (38, 238) magnetischen Kern vorstehen;
**dadurch gekennzeichnet, dass**
die erste (44, 244) und die zweite (46, 246) axiale Öffnung derart dimensioniert sind, um das chirurgische Instrument (52) aufzunehmen und die Bildung der Anastomose darin zuzulassen, und dass
der erste und der zweite Rand nahe der ersten (44, 244) und der zweiten (46, 246) axialen Öffnung des ersten (36, 236) und des zweiten (38, 238) magnetischen Kerns angeordnet sind.

2. Medizinische Vorrichtung (30, 230) nach Anspruch 1, wobei die erste (44, 244) und die zweite (46, 246) axiale Öffnung im Wesentlichen gleich groß sind.

3. Medizinische Vorrichtung (30, 230) nach Anspruch 1, wobei die erste (44, 244) und die zweite (46, 246) axiale Öffnung unterschiedlich groß sind.

4. Medizinische Vorrichtung (30, 230) nach Anspruch 3, wobei der erste (40, 240) und der zweite (42, 242) Rand so dimensioniert sind, dass sie sich ineinander verschachteln.

5. Medizinische Vorrichtung (30, 230) nach Anspruch 1, wobei der erste und der zweite Rand atraumatische, ineinandergreifende Flächen (43, 243) definieren.

6. Medizinische Vorrichtung (30, 230) nach Anspruch 1, wobei die erste (32, 232) und die zweite (34, 234) magnetische Baugruppe scheibenförmig ausgebildet sind.

7. Medizinische Vorrichtung (30, 230) nach Anspruch 1, wobei die erste (44, 244) und die zweite (46, 246) axiale Öffnung einen Durchmesser von mindestens 10 mm aufweisen.

## Revendications

1. Dispositif médical (30, 230) pour rapprocher des tissus (24, 26) de deux viscères pour la formation d'une anastomose en utilisant un instrument chirurgical (52), le dispositif médical comprenant :
un premier ensemble à aimant (32, 232) ayant un premier noyau magnétique (36, 236) définissant une première ouverture axiale (44, 244), et une première enveloppe (40, 240) ;
un second ensemble à aimant (34, 234) ayant un second noyau magnétique (38, 238) définissant une seconde ouverture axiale (46, 246), et une seconde enveloppe (42, 242) ;
au moins l'un des premier (32, 232) et second (34, 234) ensembles à aimant ayant une voie de passage transversale (48, 50, 248, 250) dimensionnée pour recevoir et permettre le passage d'un guide-fil à travers celle-ci pour le placement des premier (32, 232) et second (34, 234) ensembles à aimant ;
dans lequel les premier (32, 232) et second (34, 234) ensembles à aimant définissent des première et seconde couronnes faisant saillie axialement à partir des première (40, 240) et seconde (42, 242) enveloppes au-delà des premier (36, 236) et second (38, 238) noyaux magnétiques ;
**caractérisé en ce que**
les première (44, 244) et seconde (46, 246) ouvertures axiales sont dimensionnées pour recevoir l'instrument chirurgical (52) et permettre la formation de l'anastomose dans celles-ci, et que
les première et seconde couronnes sont positionnées à proximité des première (44, 244) et seconde (46, 246) ouvertures axiales des premier (36, 236) et second (38, 238) noyaux magnétiques.

2. Dispositif médical (30, 230) selon la revendication 1, dans lequel les première (44, 244) et seconde (46, 246) ouvertures axiales ont sensiblement la même taille.

3. Dispositif médical (30, 230) selon la revendication 1, dans lequel les première (44, 244) et seconde (46, 246) ouvertures axiales sont de différente taille.

4. Dispositif médical (30, 230) selon la revendication 3, dans lequel les première (40, 240) et seconde (42, 242) couronnes sont dimensionnées pour s'emboîter l'une dans l'autre.

5. Dispositif médical (30, 230) selon la revendication 1, dans lequel les première et seconde couronnes définissent des surfaces d'engagement atraumatiques (43, 243).

6. Dispositif médical (30, 230) selon la revendication 1, dans lequel les premier (32, 232) et second (34, 234) ensembles à aimant sont en forme de disque.

7. Dispositif médical (30, 230) selon la revendication 1, dans lequel les première (44, 244) et seconde (46, 246) ouvertures axiales ont un diamètre d' au moins 10 mm.
